# EUROPEAN PATENT SPECIFICATION

(11) **EP 0 787 483 B1**
(45) Date of publication and mention of the grant of the patent: **16.04.2003**
(21) Application number: 96203629.9
(22) Date of filing: 19.12.1996
(51) Int. Cl.: A61K 7/42

(54) **Photostable, emulsifier-free cosmetic compositions**
Photostabile, emulgatorfreie, kosmetische Mittel
Compositions cosmétiques photostables sans émulsifiant

(30) Priority: 20.12.1995 DE 19547634
(43) Date of publication of application: 06.08.1997
(73) Proprietor: Sara Lee/DE N.V., 3532 AA Utrecht (NL)
(72) Inventor: Finkel, Peter, 51519 Odenthal (DE); Voss, Eckart, 51375 Leverkusen (DE); Urban, Werner, 40764 Langenfeld (DE)
(74) Representative: Smulders, Theodorus A.H.J., Ir.

(56) References cited:
- EP-A- 0 365 370
- EP-A- 0 709 080
- EP-A- 0 717 982
- WO-A-91/11989
- WO-A-94/04131
- FR-A- 2 680 105
- FR-A- 2 695 560
- GB-A- 2 098 868
- GB-A- 2 198 944

## Description

The invention relates to photostable cosmetic compositions for protecting the human skin against skin damages caused by light, on the basis of emulsifier-free emulsions (hydrodispersion gels) for stabilizing dibenzoylmethane derivatives, in particular 1-(4-tert.butylphenyl)-3-(4-methoxyphenyl)propane-1,3-dione against photodecomposition caused by UV.

The invention further relates to the use of the compositions according to the invention for protecting the skin against UV rays and processes according to the invention for preparing said photostable compositions, wherein the UV-A screen is stabilised without destabilizing the UV-B screen.

The photostable, emulsifier-free cosmetic compositions according to the invention serve to protect the skin against UV rays and contain specific UV-A screens and specific UV-B screens.

As is known, light rays having a wavelength of from 280 nm to 400 nm lead to tanning of the human epidermis. Rays having a wavelength of from 280 nm to 320 nm, known as UV-B rays, may cause erythemas and skin burns. These UV-B rays must therefore be filtered out.

It is also known that the UV-A rays having a wavelength of from 320 nm to 400 nm, which cause the so-called direct pigmenting, may effect a change of the skin, especially in a sensitive skin or in a skin which is continually exposed the sunlight.

In particular, UV-A rays cause a loss of skin elasticity and the appearance of wrinkles, which leads to premature ageing. Moreover, they promote the onset of the erythemateous reaction or intensify this reaction in some individuals and may even be at the origin of phototoxic or photoallergic reactions. It is therefore desirable to filter out the UV-A rays.

It is known, however, that the UV-B/UV-A screen combinations commonly used in'conventional emulsions, containing fat-soluble dibenzoylmethane derivatives and in particular 1-(4-tert.butylphenyl)-3-(4-methoxyphenyl)-propane-1,3-dione, show a clear photodecomposition.

Subject to the duration and intensity of the radiation, this results in a more or less marked reduction of the protective capacity within the UV-A range during exposure of the skin to light.

DE 3 741 420 describes a light-stable cosmetic composition having improved photostability. It contains, in a cosmetic carrier having at least one fatty phase, 1-3 wt.% 4-(1,1-dimethylethyl)-4'-methoxydibenzoylmethane and at least 4.5 wt.% p-methylbenzylidenecamphor, the weight ratio of both components being more than or equal to 3.

DE 3 831 920 describes a photostable cosmetic composition containing at least 0.0025 wt.% bixin together with at least 1 wt.% of a fat-soluble UV screen, selected among 3-benzylidene-d,1-camphor and its derivatives, benzophenone derivatives and mixtures thereof in a cosmetically compatible carrier, comprising at least one fatty phase.

WO 94/04131 describes photostable sunscreen compositions containing dibenzoylmethane derivatives (UV-A absorbers) together with UV-B absorbing benzylidenecamphor compounds, in particular with inorganic pigments.

WO 93/02659 describes a photostable cosmetic formulation, containing a UV-A screen and 4-methoxybenzylidenecamphor. The UV-A screen particularly mentioned is 4(1,1-dimethylethyl)4'-methoxy-dibenzoylmethane.

WO 94/06404 describes photostable cosmetic compositions containing mixtures of UV-A screen with polymer screen on the basis of silicone-benzotriazole.

Finally, WO 91/11989 describes photostable cosmetic compositions on the basis of UV-A screens and β,β-diphenylacrylates or α-cyano-β,β-diphenylacrylates.

The photostable cosmetic compositions described in the prior art references have the'drawback that nevertheless the UV-A screen loses its activity to some extent under the action of light.

It has now been found that the photostability of the UV-A screen can be improved not only by the steps described in the above prior art references, i.e. stabilizing the UV-A screen by means of additives and combinations with other UV screens, but very essentially by means of the configuration of the composition, in particular of the inactive components of formulations.

Surprisingly, it has been found that especially emulsifier-free formulations, while abandoning derivatives of 4-methoxycinnamic acid as UV-B screen, effect a very high photostabilization of the cosmetic compositions according to the invention.

Thus, there is achieved a uniform long-lasting protection of the skin against UV-A radiation.

The invention relates to photostable cosmetic compositions for stabilizing dibenzoylmethane derivatives characterized in that they contain
a) one or more fat-soluble UV-B screens, except 4-methoxycinnamic acid and
b) dibenzoylmethane derivatives as UV-A screen in
c) an emulsifier-free oil in water (o/w) emulsion.

The present invention further relates to a process for preparing photostable cosmetic compositions according to the invention, characterized by
- first combining one or more UV screens (i.e. component I), with one or more fat components (i.e. component II), and dissolving at about 75-80 DEG C,
- then adding component IV, said component IV at least consisting of carbomer, glycerine, EDTA and water, with stirring and cooling to 30 DEG C,
- then adding component III, said component III at least consisting of perfume oil and optionally one or more preservatives,
- and finally neutralizing with a neutralizing agent (i.e. component V), and homogenizing.

The invention preferably relates to photostable cosmetic compositions containing
a) one or more fat-soluble UV-B screens of the series
   3,3,5-trimethyl-cyclohexylsalicylate,
   2-hydroxy-4-methoxybenzophenone,
   2-cyano-3,3-diphenylacrylic acid,
   salicylic acid-2-ethylhexylester,
   3-(4'-methyl)-benzylidene-bornan-2-one,
   4-isopropylbenzylsalicylate and/or
   2,4,6-trianiline-(p-carbo-2'- ethylhexyl-1'-oxy)-1,3,5-triazine,
b) 1-(4-tert.butylphenyl)-3-(4-methoxyphenyl)propane-1,3-dione as UV-A screen in
c) an emulsifier-free o/w emulsion.

Especially preferred are photostable cosmetic compositions containing
a) one or more fat-soluble UV-B screens of the series
   3,3,5-trimethyl-cyclohexylsalicylate,
   2-cyano-3, 3-diphenylacrylic acid,
   salicylic acid-2-ethylhexylester,
   3-(4'-methyl)-benzylidene-bornan-2-one and/or
   2,4,6-trianiline-(p-carbo-2'- ethylhexyl-1'-oxy)-1,3,5-triazine,
b) 1-(4-tert.butylphenyl)-3-(4-methoxyphenyl)propane-1,3-dione as UV-A screen in
c) an emulsifier-free o/w emulsion.

Besides the preferred and especially preferred UV-A or UV-B screens, the photostable cosmetic compositions according to the invention also contain water-soluble UV-A and/or water-soluble UV-B screens. The following water-soluble UV-A and/or UV-B screens are listed by way of example:
2-phenylbenzimidazole-5-sulfonic acid and their potassium, sodium and/or triethanolamino salts,
3,3'-(1,4-phenylenedimethine)-bis-(7,7-dimethyl-2-oxobicyclo-[2.2.1]-heptane-1-methanesulfonic acid) and/or their salts,
3-(4'-sulfo)benzylidene-bornan-2-one and/or their salts, 2-hydroxy-4-methoxy-benzophenone-5-sulfonic acid and/or the sodium salt.

Moreover, hydrophilic and/or hydrophobic mineral light-screen agents may be added to the photostable cosmetic compositions according to the invention. Zinc oxide (ZnO) and titanium dioxide (TiO₂) are mentioned by way of example.

The photostable cosmetic compositions according to the invention contain the above UV-A or UV-B screens in amounts usual for cosmetic applications, which amounts do not exceed corresponding admissible maximum concentrations.

The photostable cosmetic compositions according to the invention each usually contain 0.1-10 wt.% of the respective UV-A and UV-B screens to be used for the mixture in emulsifier-free o/w emulsion. Preferred are 0.5-10 wt.% of the respective UV-A and UV-B screens and especially preferred are 0.5-3 wt.% of the respective UV-A and 0.5-6 wt.% of the respective UV-B screens.

Because of the stability of the o/w emulsion, limits are imposed on the total amount of UV-A and UV-B screens, to the extent that no emulsifier is used in the photostable cosmetic compositions according to the invention.

In view of the partially liposoluble character of the screen agents used, the cosmetic compositions according to the invention contain at least one fatty phase. The compositions according to the invention may be present in the form of emulsifier-free hydrodispersion gels of liquid (milk, lotion, balm) to solid (cream) consistency. The compositions according to the invention may also be conditioned as an aerosol.

The cosmetic compositions according to the invention serve to protect the human epidermis against ultraviolet radiation and may contain cosmetic adjuvants, commonly used in such compositions. These include thickeners, softeners, humectants, surfactants, preservatives, antifoams, oils, waxes, lanolin, perfumes, propellants, colorants and/or pigments for coloring the composition itself or the skin or any other component commonly used in cosmetics, but except emulsifiers.

The compositions according to the invention may be present as an emulsion in the form of a cream or milk which, besides the combination of the UV-A screen agents and the UV-B screen agents, also contain fatty alcohols, fatty acid esters and in particular triglycerides of fatty acids, fatty acids, lanolin, natural or synthetic oils in the presence of water.

The compositions according to the invention may also be emulsions on the basis of fatty acids, oils and/or natural or synthetic waxes and contain lower alcohols, such as ethanol or glycols, such as propylene glycol and/or polyols, such as glycerine.

If the compositions according to the invention are conditioned as an aerosol, classic propellants are used, such as alkanes, fluoroalkanes and chlorofluoroalkanes.

The following examples serve to explain the invention.

### Formulation examples of hydrodispersion gels:

| | Formulation compositions | | | | |
|---|---|---|---|---|---|
| Component I, UV-screen, e.g. | 1 | 2 | 3 | 4 | 5 |
| Eusolex 6300 (UV-B screen) | 2,0 | 2,0 | 2,0 | 4,0 | 3,0 |
| Uvinul T 150 (UV-B screen) | | 1,0 | 1,0 | 2,0 | 1,5 |
| Neo Heliopan OS (UV-B screen) | | 5,0 | ./. | 3,0 | 4,0 |
| Eusolex HMS (UV-B screen) | | ./. | 5,0 | ./. | |
| Neo Heliopan 303 (UV-B screen) | | | 3,0 | | 4,0 |
| Parsol 1789 (UV-A screen) | 2,0 | 2,0 | 2,0 | 3,0 | 2,0 |

| Component II, fat components e.g. | 1 | 2 | 3 | 4 | 5 |
|---|---|---|---|---|---|
| Finsolv TN | 10,0 | | 10,0 | 12,0 | 12,0 |
| Mugliol 812 | | 5,0 | | 2,0 | |
| Cetiol 868 etc. | | 5,0 | | 3,0 | |

| Component III | 1 | 2 | 3 | 4 | 5 |
|---|---|---|---|---|---|
| Perfume oil Preservative | 0,4 q.s. | 0,4 q.s. | 0,4 q.s. | 0,4 q.s. | 0,4 q.s. |
| Component IV | 1 | 2 | 3 | 4 | 5 |
| Carbomer | 0,4 | 0,4 | 0,4 | 0,4 | 0,8 |
| Glycerine | 4,0 | 3,0 | 3,0 | 3,0 | 3,0 |
| EDTA | 0,1 | 0,1 | 0,1 | 0,1 | 0,1 |
| Water | to 100,0 | to 100,0 to 100,0 | | | |

| Component V | 1 | 2 | 3 | 4 | 5 |
|---|---|---|---|---|---|
| Water | | | | | 20,0 |
| Novantiolic acid | | | | | 2,0 |
| (water soluble UV-B screen) | | | | | |
| Neutralizing agent e.g. | q.s. | q.s. | q.s. | q.s. | q.s. |
| (NaOH, Triethanolamine) | | | | | |

### Preparation Formulation Examples 1-4

Components I and II are combined and dissolved at about 75-80°C. Component IV is dissolved and dispersed. Combination of I/II and IV with stirring, cooling to about 30°C. Addition of Component III with stirring. Neutralization with Component IV, followed by homogenization.

### Preparation Formulation Example 5

Components I and II are combined and dissolved at about 70-80°C. Component IV is dissolved and dispersed. Component V is dissolved.

Combination of I/II and IV with stirring, cooling to about 30°C. Addition of Component III and Component V with vigorous stirring, optionally adjustment of the pH value with neutralizing agent, followed by homogenization.

The enclosed illustration shows the results of an in vitro examination in which the reduction of the protective capacity is detected as a relative reduction of the in vitro light-screen factor SPF (Modified Method according to Diffey, B.; J.ö. Soc. Cosmet. Chem. 1989, 40, 127-133) and as a change of the ratio of UV-A to UV-B protective capacity (UV-A/UV-B). To this end, the product was placed on a roughened quartz glass plate with about 1 mg/cm² and irradiated. The time of irradiation occurred at an energy equivalent of 0 MED to 10 MED (MED = Minimal Erytherm Dosis).

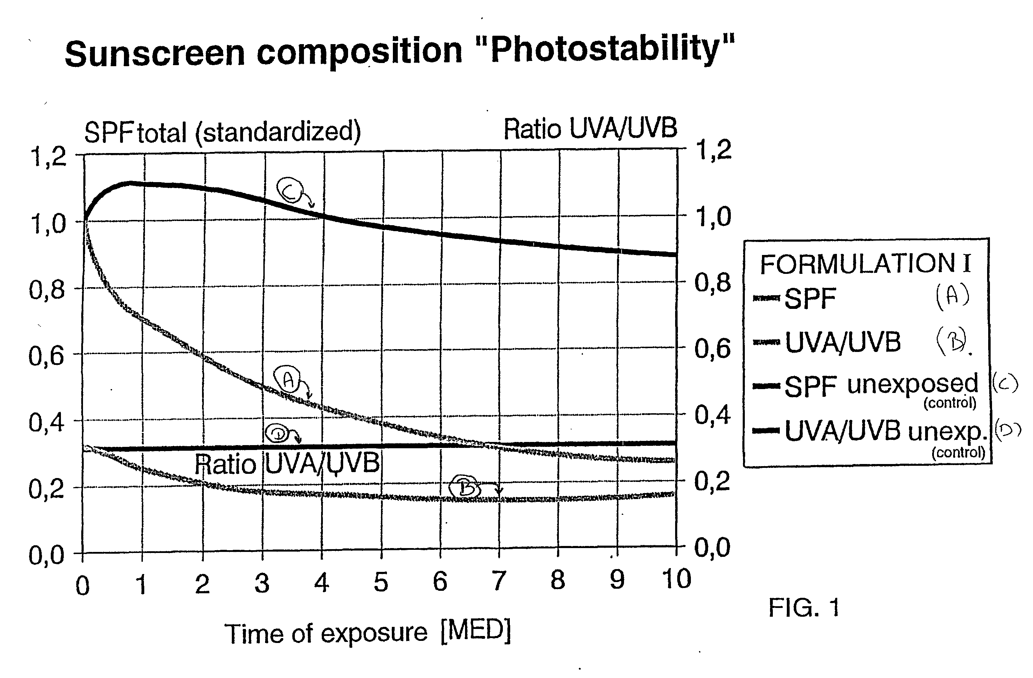

Figure 1 shows a usual sunscreen emulsion on the basis of 1-(4-tert.butylphenyl)-3-(4-methoxyphenyl)-propane-1,3-dione and 4-methoxycinnamic acid. Figure 2 (not shown) shows an emulsion stabilized by 3-(4'-methyl)benzylidene-)bornan-2-one, as it corresponds with a formulation from DE 37 41 420. Figure 3 (not shown) shows an emulsifier-free composition according to the invention. The formulation according to the invention may be considered fully stable over the total time of irradiation.

## Claims

1. Emulsifier-free photostable cosmetic compositions for stabilizing dibenzoylmethane derivatives, **characterized in that** they contain
a) one or more fat-soluble UV-B screens, except
4-methoxycinnamic acid,
b) dibenzoylmethane derivatives as UV-A screen in
c) an emulsifier-free o/w emulsion

2. Photostable cosmetic compositions according to claim 1, **characterized in that** they contain
a) one or more fat-soluble UV-B screens of the series
3,3,5-trimethyl-cyclohexylsalicylate,
2-hydroxy-4-methoxybenzophenone,
2-cyano-3,3-diphenylacrylic acid,
salicylic acid-2-ethylhexylester,
3-(4'-methyl)-benzylidene-bornan-2-one,
4-isopropylbenzylsalicylate or
2,4,6-trianiline-(p-carbo-2'-ethylhexyl-1'-oxy)-1,3,5-triazine,
b) 1-(4-tert.butylphenyl)-3-(4-methoxyphenyl)propane-1,3-dione as UV-A screen in
c) an emulsifier-free o/w emulsion.

3. Photostable cosmetic compositions according to claim 1 or 2, **characterized in that** they contain
a) one or more fat-soluble UV-B screens of the series
3,3,5-trimethyl-cyclohexylsalicylate,
2-cyano-3,3-diphenylacrylic acid,
salicylic acid-2-ethylhexylester,
3-(4'-methyl)-benzylidene-bornan-2-one or
2,4,6-trianiline-(p-carbo-2'-ethylhexyl-1'-oxy)-1,3,5-triazine,
b) 1-(4-tert.butylphenyl)-3-(4-methoxyphenyl)propane-1,3-dione as UV-A screen in
c) an emulsifier-free o/w emulsion.

4. Photostable cosmetic compositions according to claims 1-3, **characterized in that** they additionally contain water-soluble UV-A and/or UV-B screens of the series
2-phenylbenzimidazole-5-sulfonic acid and their potassium, sodium and/or triethanolamino salts,
3,3'-(1,4-phenylenedimethine)-bis-(7,7-dimethyl-2-oxobicyclo-[2.2.1]-heptane-1-methanesulfonic acid) and their salts,
3-(4'-sulfo)benzylidene-bornan-2-one and their salts or
2-hydroxy-4-methoxy-benzophenone-5-sulfonic acid and/or the sodium salt thereof.

5. Photostable cosmetic compositions according to claims 1-4, **characterized in that** they contain ZnO and/or TiO₂ as hydrophilic and/or hydrophobic mineral light-screen agents.

6. Photostable cosmetic compositions according to claims 1-5, **characterized in that** they contain the respective UV-A and UV-B screens to be used for the mixture in amounts of 0.1-10 wt.%.

7. The use of the photostable cosmetic compositions according to claims 1-6, for protecting the human skin against skin damages caused by light.

8. A process for preparing the photostable cosmetic compositions according to any of the previous claims, **characterized by**
- first combining one or more UV screens (i.e. component I), with one or more fat components (i.e. component II), and dissolving at about 75-80 DEG C,
- then adding component IV, said component IV at least consisting of carbomer, glycerine, EDTA and water, with stirring and cooling to 30 DEG C,
- then adding component III, said component III at least consisting of perfume oil and optionally one or more preservatives,
- and finally neutralizing with a neutralizing agent (i.e. component V), and homogenizing.

## Patentansprüche

1. Emulgatorfreie photostabile kosmetische Zusammensetzungen zur Stabilisierung von Dibenzoylmethan-Derivaten, **dadurch gekennzeichnet, dass** sie enthalten:
a) ein oder mehrere fettlösliche UV-B-Fliter, ausgenommen 4-Methoxyzimtsäure,
b) Dibenzoylmethan-Derivate als UV-A-Filter in
c) einer emulgatorfrelen o/w-Emulsion.

2. Photostabile kosmetische Zusammensetzungen nach Anspruch 1, **dadurch gekennzeichnet, dass** sie enthalten:
a) ein oder mehrere fettlösliche UV-B-Filter der Reihe
3,3,5-Trimethyl-cyclohexylsalicylat,
2-Hydroxy-4-methoxybenzophenon,
2-Cyano-3,3-diphenylacrylsäure,
Salicylsäure-2-ethylhexylester,
3-(4'-Methyl)-benzyliden-bornan-2-on,
4-Isopropylbenzylsalicylat oder
2,4,6-Trianilin-(p-carbo-2-'ethylheyl-1'-oxy)-1,3,5-triazin,
b) 1-(4-tert.-Butylphenyl)-3-(4-methoxyphenyl)propan-1,3-dion als UV-A-Filter in
c) einer emulgatorfreien o/w-Emulsion.

3. Photostabile kosmetische Zusammensetzungen nach Anspruch 1 oder 2, **dadurch gekennzeichnet, dass** sie enthalten:
a) einen oder mehrere fettlösliche UV-B-Filter der Reihe:
3,3,5-Trimethyl-cyclohexylsalicylat
2-Cyano-3,3-diphenylacrylsäure,
Salicylsäure-2-ethylhexylester,
3-(4'-Methyl)-benzyliden-bornan-2-on oder
2,4,6-Trianilin-(p-carbo-2-'ethylheyl-1'-oxy)-1,3,5-triazin,
b) 1-(4-tert.-Butylphenyl)-3-(4-methoxyphenyl)propan-1,3-dion als UV-A-Filter in
c) einer emulgatorfreien o/w-Emulsion.

4. Photostabile kosmetische Zusammensetzungen nach den Ansprüchen 1 bis 3, **dadurch gekennzeichnet, dass** sie zusätzlich wasserlösliche UV-A- und/oder UV-B-Filter der Reihe:
2-Phenylbenzimidazol-5-sulfonsäure und deren Kalium-, Natriumund/oder Triethanolamin-Salze,
3,3'-(1,4-Phenylendimethin)-bis-(7,7,dimethyl-2-oxobicyclo-[2.2.1]-heptan-1-methansulfonsäure) und deren Salze,
3-(4'-Sulfo)benzyliden-bornan-2-on und deren Salze oder
2-Hydroxy-4-methoxy-benzophenon-5-sulfonsäure und/oder das Natriumsalz davon,
enthalten.

5. Photostabile kosmetische Zusammensetzungen nach den Ansprüchen 1 - 4, **dadurch gekennzeichnet, dass** sie ZnO und/oder TiO₂ als hydrophile und/oder hydrophobe mineralische Lichtschutzmittel enthalten.

6. Photostabile kosmetische Zusammensetzungen nach den Ansprüchen 1 - 5, **dadurch gekennzeichnet, dass** sie die jeweiligen UV-Aund UV-B-Filter, die in der Mischung verwendet werden, in Mengen von 0,1 - 10 Gew.-% enthalten.

7. Verwendung der photostabilen kosmetischen Zusammensetzungen nach Ansprüchen 1 - 6 zum Schutz der menschlichen Haut gegen Hautschäden, die durch Licht verursacht werden.

8. Verfahren zur Herstellung der photostabilen kosmetischen Zusammensetzungen nach irgend einem der vorstehenden Patentansprüche, **gekennzeichnet durch**:
- erstens, Kombinieren eines oder mehrerer UV-Filter (d.h. Komponente I) mit einer oder mehreren Fett-Komponenten (d.h. Komponente II) und Lösen bei ungefähr 75 bis 80°C,
- anschließend Zugeben der Komponente IV, wobei die genannte Komponente IV zumindest teilweise besteht aus Carbomer, Glycerin, EDTA und Wasser, unter Rühren und Kühlen auf 30°C,
- anschließend Zugeben der Komponente III, wobei die genannte Komponente III mindestens teilweise aus Parfumölen und wahlweise einem oder mehreren Konservierungsmitteln besteht, und
- anschließendes Neutralisieren mit einem Neutrallslerungsmittel (d.h. Komponente V) und Homogenisieren.

## Revendications

1. Compositions cosmétiques stables à la lumière sans émulsifiant de stabilisation de dérivés de dibenzoylméthane, **caractérisées en ce qu'**elles contiennent
a) un ou plusieurs écrans UV-B liposolubles, hormis l'acide 4-méthoxycinnamique,
b) des dérivés de dibenzoylméthane en tant qu'écran UV-A dans
c) une émulsion huile/eau sans émulsifiant.

2. Compositions cosmétiques stables à la lumière selon la revendication 1, **caractérisées en ce qu'**elles contiennent
a) un ou plusieurs écrans UV-B liposolubles de la série
salicylate de 3,3,5-triméthylcyclohexyle,
2-hydroxy-4-méthoxybenzophénone,
acide 2-cyano-3,3-diphénylacrylique,
salicylate de 2-éthylhexyle,
3-(4'-méthyl)-benzylidène-bornan-2-one,
salicylate de 4-isopropylbenzyle ou
2,4,6-trianiline-(p-carbo-2'-éthylhexyl-1'-oxy)-1,3,5-triazine,
b) 1-(4-tert.butylphényl)-3-(4-méthoxyphényl)propane-1,3-dione en tant qu'écran UV-A dans
c) une émulsion huile/eau sans émulsifiant.

3. Compositions cosmétiques stables à la lumière selon la revendication 1 ou 2, **caractérisées en ce qu'**elles contiennent
a) un ou plusieurs écrans UV-B liposolubles de la série
salicylate de 3,3,5-triméthylcyclohexyle,
acide 2-cyano-3,3-diphénylacrylique,
salicylate de 2-éthylhexyle,
3-(4'-méthyl)-benzylidène-bornan-2-one, ou
2,4,6-trianiline-(p-carbo-2'-éthylhexyl-1'-oxy)-1,3,5-triazine,
b) 1-(4-tert.butylphényl)-3-(4-méthoxyphényl)propane-1,3-dione en tant qu'écran UV-A
dans
c) une émulsion huile/eau sans émulsifiant.

4. Compositions cosmétiques stables à la lumière selon les revendications 1 à 3, **caractérisées en ce qu'**elles contiennent en outre des écrans UV-A et/ou UV/B hydrosolubles de la série
acide 2-phénylbenzimidazole-5-sulfonique et ses sels de potassium, de sodium et/ou de triéthanolamino,
acide 3,3'-(1,4-phénylènediméthyne)-bis-(7,7-diméthyl-2-oxobicyclo-[2.2.1]-heptane-1-méthanesulfonique et ses sels,
3-(4'-sulfo)benzylidène-bornan-2-one et ses sels ou acide 2-hydroxy-4-méthoxy-benzophénone-5-sulfonique et/ou le sel de sodium correspondant.

5. Compositions cosmétiques stables à la lumière selon les revendications 1 à 4, **caractérisées en ce qu'**elles contiennent ZnO et/ou TiO₂ en tant qu'agents écrans minéraux contre la lumière hydrophiles et/ou hydrophobes.

6. Compositions cosmétiques stables à la lumière selon les revendications 1 à 5, **caractérisées en ce qu'**elles contiennent les écrans UV-A et UV-B respectifs à utiliser dans le mélange en quantités de 0,1 à 10% en poids.

7. Utilisation des compositions cosmétiques stables à la lumière selon les revendications 1 à 6, pour protéger la peau humaine contre les dommages de la peau provoqués par la lumière.

8. Procédé de préparation des compositions cosmétiques stables à la lumière selon l'une quelconque des revendications précédentes, **caractérisé par**
- tout d'abord la combinaison d'un ou de plusieurs écrans UV (c'est-à-dire le composant I), avec un ou plusieurs composants gras (c'est-à-dire le composant II), et dissolution à une température d'environ 75 à 80°C,
- puis l'ajout d'un composant IV, ledit composant IV étant constitué au moins par un carbomère, la glycérine, l'EDTA et l'eau, avec une agitation et un refroidissement jusqu'à 30°C,
- puis l'ajout d'un composant III, ledit composant III étant constitué au moins par une huile de parfum et éventuellement d'un ou de plusieurs conservateurs,
- et enfin la neutralisation par un agent neutralisant (c'est-à-dire le composant V), et l'homogénéisation.
